# EUROPEAN PATENT APPLICATION

(11) **EP 4 800 384 A1**
(43) Date of publication of application: **02.09.2026**
(21) Application number: 24882754.5
(22) Date of filing: 21.10.2024
(51) Int. Cl.: G01N 27/22, G06N 3/08

(54) **ELECTRONIC DEVICE AND METHOD FOR PERFORMING ANTIBIOTIC SUSCEPTIBILITY TEST OF MICROORGANISM**

(30) Priority: 26.10.2023 KR 20230144395
(71) Applicant: Protia Inc., Seoul 07528 (KR)
(72) Inventor: LIM, Kook Jin, Seoul 07324 (KR); CHOI, Yon-Sik, Seoul 06522 (KR); SHIN, Seok Kyo, Gimpo-si, Gyeonggi-do 10066 (KR); KIM, Tae Young, Seoul 07517 (KR)
(74) Representative: J A Kemp LLP
(86) International application number: PCT/KR2024/015975
(87) International publication number: WO 2025/089727

(57) **Abstract**

An electronic device for performing an antibiotic susceptibility test of a microorganism, according to one embodiment of the present invention, may comprise a processor, which: obtains antibiotic data obtained by measuring the change in electrical characteristics according to antibiotic treatment of a target microorganism; inputs, into a deep learning model trained to determine whether the antibiotic data is positive or negative by using positive control group data, negative control group data and antibiotic data of the microorganism, the positive control group data, the negative control group data and the antibiotic data of the target microorganism, thereby determining whether the antibiotic data of the target microorganism is positive or negative; and uses the determination result so as to identify antibiotic susceptibility information about the target microorganism according to antibiotic type and concentration.

## Description

### TECHNICAL FIELD

The present invention relates to an electronic device and method for performing an antibiotic susceptibility test of microorganisms.

### BACKGROUND ART

Among various diseases that occur in the human body, there are diseases caused by microbial infections, including urinary tract infections and sepsis. In order to treat these diseases caused by microbial infections, antibiotics that suppress the growth of microorganisms are needed, and antibiotics suppress the growth of microorganisms or kill microorganisms. However, more than 100 types of antibiotics have been developed so far, and their effectiveness varies depending on the type of microorganism or whether the microorganism is resistant to antibiotics. Therefore, the most effective antibiotic should be selected for each patient, and the diagnostic test method therefor is called an antibiotic susceptibility test.

One of the antibiotic susceptibility tests is a method of conducting a test using changes in electrical characteristics according to the presence or absence of microbial growth. In this case, data representing changes in electrical characteristics, such as a capacitance graph, are analyzed by principal component analysis (PCA) to quantify the similarity with the control group.

However, in the differential graph obtained during the PCA process, when there was a difference in data values between the time-dependent electric capacity graph of a control group and the time-dependent electric capacity graph of an antibiotic-treated microorganism, many errors occurred that were judged differently from the actual results. For example, there may be a difference in the time at which the peak value of the electric capacity appears or in the peak value between the time-dependent electric capacity graph of the control group and the time-dependent electric capacity graph of antibiotic-treated microorganisms.

Therefore, there is a need to find a new analysis method that reduces judgment errors while using data measuring electrical characteristic changes.

### DISCLOSURE

### TECHNICAL PROBLEM

An object of the present invention is to provide an electronic device and method for determining whether a microorganism is susceptible to antibiotics, which can significantly reduce determination errors.

### TECHNICAL SOLUTION

An electronic device for performing an antibiotic susceptibility test of a microorganism according to an embodiment of the present invention may include a processor configured to: obtain antibiotic data obtained by measuring changes in electrical characteristics according to antibiotic treatment of a target microorganism; input positive control group data, negative control group data and antibiotic data of the target microorganism into a deep learning model trained to determine whether the antibiotic data is positive or negative by using positive control group data, negative control group data and antibiotic data of a microorganism, thereby determining whether antibiotic data of the target microorganism is positive or negative; and use the determination result so as to identify antibiotic susceptibility information about the target microorganism according to antibiotic type and concentration.

The deep learning model may extract feature vectors of positive control group data, negative control group data and antibiotic data of a microorganism, respectively, and learn to make feature vectors of the antibiotic data similar to feature vectors of the negative control group data or the positive control group data based on labeling of the antibiotic data as positive or negative.

The processor may extract feature vectors of positive control group data, negative control group data and antibiotic data of the target microorganism, measure similarity between feature vectors of the antibiotic data and feature vectors of the positive control group data and the negative control group data by using the deep learning model, and determine whether antibiotic data of the target microorganism is positive or negative based on the similarity.

The processor may obtain the antibiotic data through a biosensor including a plurality of cross-electrodes that are implemented to measure in real time changes in electrical characteristics according to the proliferation of a microorganism in response to antibiotic treatment.

The processor may calculate a minimum inhibitory concentration (MIC) for each antibiotic type of the target microorganism by matching antibiotic type and concentration information corresponding to each of the plurality of cross-electrodes with the determination result.

The processor may determine any one of susceptibility, intermediate resistance and resistance of the target microorganism to each antibiotic type based on the minimum inhibitory concentration, thereby obtaining the antibiotic susceptibility information.

A method for testing antibiotic susceptibility of a microorganism performed by an electronic device according to an embodiment of the present invention may include obtaining antibiotic data by measuring changes in electrical characteristics of a target microorganism according to antibiotic treatment; inputting positive control group data, negative control group data and antibiotic data of the target microorganism into a deep learning model trained to determine whether antibiotic data of a microorganism is positive or negative by using positive control group data, negative control group data and antibiotic data of the microorganism, thereby determining whether antibiotic data of the target microorganism is positive or negative; and using the determination result so as to identify antibiotic susceptibility information about the target microorganism according to an antibiotic type and concentration.

### ADVANTAGEOUS EFFECT

According to one embodiment of the present invention, since it is possible to accurately determine whether there is susceptibility according to the antibiotic concentration, the accuracy of susceptibility test for each type of antibiotic is dramatically increased.

According to one embodiment of the present invention, since a deep learning model is trained by using feature vectors of data, it is possible to accurately determine whether it is positive or negative without being affected by differences in time-series electrical characteristic changes between antibiotic data and control group data.

According to one embodiment of the present invention, by using a deep learning model trained to make feature vectors similar between data, data whose time-series electrical characteristic changes are unclear can be more accurately determined as positive or negative.

### DESCRIPTION OF THE DRAWINGS

FIG. 1 is a schematic diagram showing an antibiotic susceptibility testing system according to one embodiment of the present invention.
FIG. 2 is a block diagram showing the configuration of an electronic device according to one embodiment of the present invention.
FIG. 3 is a drawing showing the operation flow diagram of an electronic device according to one embodiment of the present invention.
FIG. 4 is a drawing showing a biosensor according to one embodiment of the present invention.
FIG. 5 is a diagram showing the learning process of a deep learning model according to one embodiment of the present invention.
FIG. 6 is a drawing showing the operation flow diagram of an electronic device according to a first embodiment of the present invention.
FIG. 7 is a drawing showing the operation flow diagram of an electronic device according to a second embodiment of the present invention.
FIG. 8 is a diagram showing performance criteria for an antibiotic susceptibility test according to one embodiment of the present invention.

### MODES FOR INVENTION

Hereinafter, preferred embodiments of the present invention will be described in detail with reference to the accompanying drawings. The detailed description to be disclosed below together with the accompanying drawings is intended to describe exemplary embodiments of the present invention, and is not intended to represent the only embodiments in which the present invention may be practiced. In the drawings, in order to clearly describe the present invention, parts that are not related to the description may be omitted, and the same reference numerals may be used for the same or similar components throughout the specification.

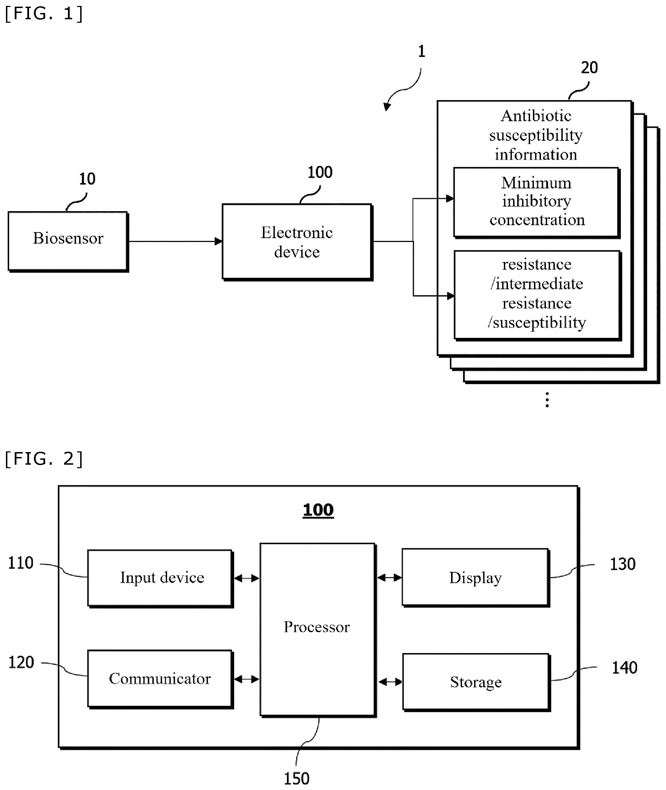

FIG. 1 is a schematic diagram showing an antibiotic susceptibility testing system according to one embodiment of the present invention.

An antibiotic susceptibility test system 1 (hereinafter, also referred to as system 1) according to one embodiment of the present invention may include a biosensor 10 and an electronic device 100.

A biosensor 10 according to one embodiment of the present invention is a sensor that measures changes in electrical characteristics, and may be composed of a plurality of interdigitated electrodes (IDEs). The biosensor 10 may be implemented to measure changes in electrical characteristics in real time according to whether microorganisms proliferate by treating microorganisms with antibiotics at each interdigitated electrode.

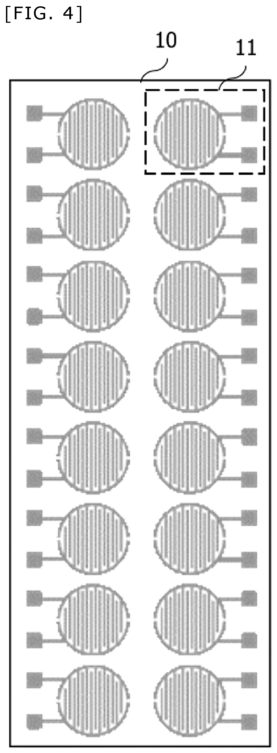

In this case, the electrical characteristics may be capacitance, impedance, resistance, reactance and the like. Therefore, when measuring changes in electrical characteristics, there is an advantage in that sensitivity may be increased even with a lower concentration and a smaller amount of sample compared to when measuring changes in optical characteristics. The biosensor 10 may measure according to the type and concentration of antibiotics treated to the target microorganism, and an example of the biosensor 10 is illustrated in FIG. 4.

An electronic device 100 according to one embodiment of the present invention is a device that performs an antibiotic susceptibility test of microorganisms, and may be implemented as a computer, a server, a smart phone, a tablet PC, a smart pad, a laptop and the like.

The electronic device 100 may use antibiotic data received from the biosensor 10 to calculate a minimum inhibitory concentration (MIC) for each type of antibiotic for the target microorganism and perform a susceptibility test. The minimum inhibitory concentration refers to the lowest concentration of the antibiotic required to inhibit visible growth of the microorganism.

The results of an antibiotic susceptibility test may be divided into, for example, resistance (R), intermediate resistance (I) and susceptibility (S). For example, in order to treat an infection caused by a microorganism, an antibiotic that is susceptible to the microorganism must be used, and it means that a microorganism that is determined as susceptible may be treated by prescribing an antibiotic at the recommended dosage for the microorganism and the infected site. In addition, a microorganism determined as showing intermediate resistance means that the minimum inhibitory concentration of the antibiotic for the target microorganism is similar to the maximum concentration of the drug that can be prescribed, which may reduce the therapeutic effect. Meanwhile, a microorganism determined as showing resistance may not be treated with the maximum concentration of the drug that can be prescribed.

As described above, when performing a susceptibility test by analyzing antibiotic data using principal components, there were cases where the data values differed despite the graph pattern being similar to that of the control group, and the results were determined differently from the actual results.

In the present invention, a method for performing a susceptibility test through the pattern analysis of time series of antibiotic data using a deep learning model is proposed.

Hereinafter, the configuration and operation of an electronic device according to one embodiment of the present invention will be specifically described with reference to the drawings.

FIG. 2 is a block diagram showing the configuration of an electronic device according to one embodiment of the present invention.

An electronic device 100 according to one embodiment of the present invention may include an input device 110, a communicator 120, a display 130, a storage 140 and a processor 150.

The input device 110 generates input data in response to a user input of the electronic device 100. For example, the user input may be a user input for starting the operation of the electronic device 100, a user input for obtaining antibiotic data, a user input for labeling antibiotic data for learning, a user input for inputting the type and concentration of antibiotics to be processed for each cross-electrode of the biosensor 10 and the like, and additionally, if it is a user input required in the process of performing deep learning model training, antibiotic susceptibility testing and the like, it may be applied without limitation.

The input device 110 includes at least one input means. The input device 110 may include a keyboard, a key pad, a dome switch, a touch panel, a touch key, a mouse, a menu button and the like.

The communicator 120 may perform communication with external devices such as a biosensor 10 and a server to transmit and receive antibiotic data, positive control group data, negative control group data, antibiotic type and concentration information, antibiotic susceptibility information, deep learning models and the like.

To this end, the communicator 120 may perform wireless communication such as 5G (5th generation communication), LTE-A (long term evolution-advanced), LTE (long term evolution), Wi-Fi (wireless fidelity) and Bluetooth, or wired communication such as LAN (local area network), WAN (Wide Area Network) and power line communication.

The display 130 displays display data according to the operation of the electronic device 100. The display 130 may display a screen displaying at least one of antibiotic data, positive control group data and negative control group data, a screen displaying antibiotic type and concentration information, a screen displaying antibiotic susceptibility information, a screen receiving user input and the like.

The display 130 includes a liquid crystal display (LCD), a light emitting diode (LED) display, an organic light emitting diode (OLED) display, a micro electro mechanical systems (MEMS) display and an electronic paper display. The display 130 may be implemented as a touch screen by being combined with the input device 110.

The storage 140 may store operation programs of the electronic device 100. The storage 140 may include non-volatile storage that is capable of preserving data (information) regardless of whether power is supplied, and volatile memory into which data to be processed by the processor 150 is loaded and which cannot preserve data if power is not supplied. The storage includes flash memory, hard-disc drive (HDD), solid-state drive (SSD), ROM (Read Only Memory) and the like, and the memory includes buffer, RAM (Random Access Memory) and the like.

The storage 140 may store antibiotic data, positive control group data, negative control group data, antibiotic type and concentration information, antibiotic susceptibility information, deep learning models and the like. The storage 140 may store computational programs and the like that are required in the process of obtaining data, determining positive/negative antibiotic data of target microorganisms, identifying antibiotic susceptibility information, extracting feature vectors from time series data, measuring similarity between feature vectors and the like.

The processor 150 may control at least one other component (e.g., hardware or software component) of the electronic device 100 by executing software such as a program, and may perform various data processing or operations.

The processor 150 according to one embodiment of the present invention obtains antibiotic data by measuring changes in electrical characteristics according to antibiotic treatment of a target microorganism, inputs positive control group data, negative control group data and antibiotic data of the target microorganism into a deep learning model trained to determine whether the antibiotic data is positive or negative by using positive control group data, negative control group data, and antibiotic data of a microorganism, thereby determining whether the antibiotic data of the target microorganism is positive or negative, and using the determination result to identify antibiotic susceptibility information of the target microorganism according to the type and concentration of the antibiotic.

The processor 150 according to one embodiment of the present invention learns a deep learning model that determines whether the antibiotic data is positive or negative by using positive control group data, negative control group data and antibiotic data of microorganisms, or uses by receiving and storing a deep learning model generated through pre-learning from the outside, but is not limited to any one.

Meanwhile, the processor 150 may perform at least some of data analysis, processing and result information generation for performing the above operations by using at least one of a machine learning, neural network or deep learning algorithm as a rule-based or artificial intelligence algorithm. Examples of the neural network may include models such as a CNN (Convolutional Neural Network), a DNN (Deep Neural Network), an RNN (Recurrent Neural Network) and a Vision Transformer.

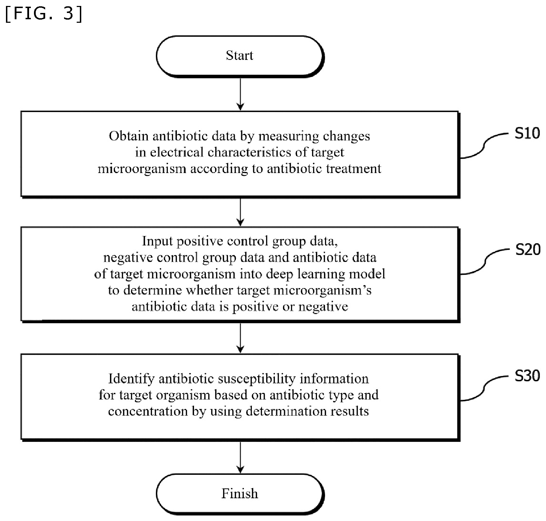

FIG. 3 is a drawing showing the operation flow diagram of an electronic device according to one embodiment of the present invention.

According to one embodiment of the present invention, the processor 150 may obtain antibiotic data measuring changes in electrical characteristics of target microorganisms according to antibiotic treatment S10.

The target microorganism may be bacteria, and the bacteria are preferably, but not limited to, gram-positive bacteria, gram-negative bacteria and antibiotic-resistant strains thereof. Specifically, the gram-positive bacteria may be any one or more selected from the group consisting of *Bacillus subtilis*, *Staphylococcus aureus*, *Enterococcus faecalis* and *Staphylococcus epidermidis*, and the gram-negative bacteria may be any one or more selected from the group consisting of *Escherichia coli*, *Psedomonas aeruginosa*, *Acinetobacter baumannii* and *Salmonella typhimurium*.

The antibiotic is not specifically limited in type, and any antibiotic whose susceptibility can be measured by the biosensor 10 according to the present invention is included. Specifically, the antibiotic may be selected from the group consisting of Ampicillin, Tetracycline, Gentamicin, Erythromycin, Vancomycin, Linezolid, Methicillin, Oxacillin, Cefotaxime, Rifampicin, Amikacin, Amikacin, Kanamycin, Tobramycin, Neomycin, Ertapenem, Doripenem, Imipenem/Cilastatin, Meropenem, Ceftazidime, Cefepime, Ceftaroline, Ceftobiprole, Aztreonam, Piperacillin, Polymyxin B, Colistin, Ciprofloxacin, Levofloxacin, Moxifloxacin, Gatifloxacin, Tigecycline, combinations thereof and derivatives thereof, and preferably, it may be Ampicillin or Tetracycline, but is not limited thereto.

Antibiotic data refers to data measuring changes in electrical characteristics over time after antibiotic treatment of microorganisms. For example, if the microorganism is resistant to an antibiotic to which it was treated, the antibiotic data will show an increase in capacitance over time as the bacteria proliferate. On the other hand, if the microorganism is susceptible to an antibiotic to which it was treated, the antibiotic will suppress the proliferation of the bacteria or kill the bacteria, and thus, there will be no change in bacterial concentration, and the antibiotic data will show a constant electrical capacity over time.

The processor 150 may obtain antibiotic data from the biosensor 10, but is not limited thereto, and the acquisition path is not limited to any one, such as receiving antibiotic data from an external device that receives the data through the biosensor 10.

According to one embodiment of the present invention, the processor 150 inputs positive control group data, negative control group data and antibiotic data of a target microorganism into a deep learning model trained to determine whether antibiotic data is positive or negative by using positive control group data, negative control group data and antibiotic data of a microorganism, thereby determining whether antibiotic data of the target microorganism is positive or negative S20.

Positive control group data refers to data measuring changes in electrical characteristics over time by growing only microorganisms without antibiotics, and negative control group data refers to data measuring changes in electrical characteristics over time in a culture medium (a liquid or solid material used for growing and preserving microorganisms) without microorganisms.

Qualitatively, if the antibiotic data is similar to the positive control group data, the microorganism is likely to be resistant to the antibiotic, and if it is similar to the negative control group data, the microorganism is likely to be susceptible to the antibiotic.

In the present invention, a deep learning model trained to determine whether antibiotic data is positive or negative is used by utilizing the time-series characteristics of these data. Through this, it is possible to quantitatively analyze whether antibiotic data is similar to positive control group data or negative control group data. In this case, if antibiotic data is similar to positive control group data, it is determined as positive (P), and if it is similar to negative control group data, it is determined as negative (N).

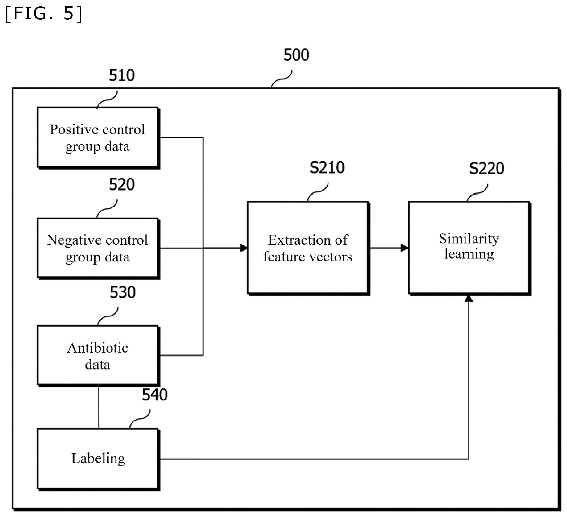

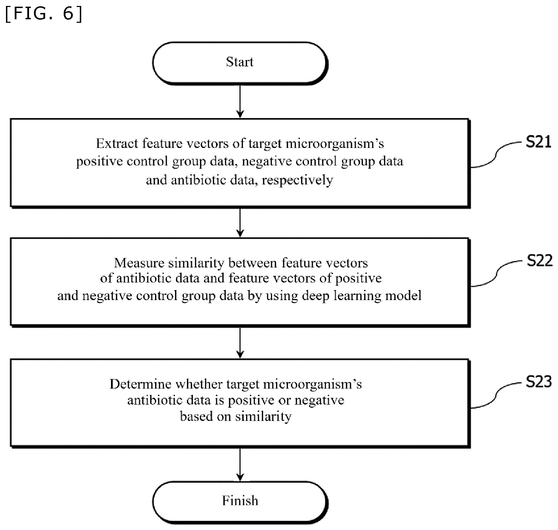

The specific learning process of the deep learning model is explained with reference to FIG. 5, and the determination process using the deep learning model is explained with reference to FIG. 6.

According to one embodiment of the present invention, the processor 150 may identify antibiotic susceptibility information of a target microorganism according to the type and concentration of antibiotics using the determination result S30.

The processor 150 may obtain a determination result that determines whether the antibiotic data of the target microorganism is positive or negative. In this case, the determination result is obtained in response to a case where a specific antibiotic is treated at a specific concentration to the target microorganism, and the processor 150 may obtain a determination result according to the antibiotic concentration for each type of antibiotic. The processor 150 may synthesize the determination results to calculate a minimum inhibitory concentration for each type of antibiotic for the target microorganism.

The processor 150 may obtain antibiotic susceptibility information by determining any one of susceptibility, intermediate resistance and resistance for each antibiotic type of the target microorganism based on the minimum inhibitory concentration. In this case, the processor 150 may determine any one of susceptibility, intermediate resistance and resistance for each antibiotic type by applying the antibiotic susceptibility test performance standards (e.g., CLSI guidelines, etc.) of the target microorganism.

According to one embodiment of the present invention, since it is possible to accurately determine whether there is susceptibility according to the antibiotic concentration, the accuracy of susceptibility testing for each type of antibiotic is dramatically increased.

FIG. 4 is a drawing showing a biosensor according to one embodiment of the present invention.

A biosensor 10 according to one embodiment of the present invention is provided with a plurality of cross-electrodes 11, and may further include a receptacle (not shown) capable of storing the cross-electrodes 11, antibiotics and microorganisms inside. Specifically, when measuring the antibiotic susceptibility of microorganisms using the biosensor 10, microorganisms or antibiotics may be processed inside the storage. The storage may be of an open top type.

FIG. 5 is a diagram showing the learning process of a deep learning model according to one embodiment of the present invention.

As previously explained with reference to S20 of FIG. 3, a deep learning model 500 may be used to determine whether antibiotic data of a target microorganism is positive or negative. The following description assumes that the processor 150 trains the deep learning model 500.

The deep learning model 500 may utilize frameworks such as, for example, SVM (Support Vector Machine), LSTM (Long Short-term Model), Transformer and Recurrent Neural Network (RNN), but is not limited to any one.

First of all, the processor 150 may extract feature vectors of positive control group data 510, negative control group data 520 and antibiotic data 530 prepared for learning S210, respectively.

In this case, the positive control group data 510, the negative control group data 520 and the antibiotic data 530 are configured as one set prepared under the same conditions (same microorganism, same culture medium, same processing method, etc.). In addition, the antibiotic data 530 is prepared with the correct answer as to whether it is positive or negative, that is, labeling 540.

The processor 150 may train a deep learning model 500 such that the feature vectors of the antibiotic data become similar to the feature vectors of the positive control group data 510 or the feature vectors of the negative control group data 520 based on the labeling 540 of the positive or negative antibiotic data S220.

For example, in the case of antibiotic data 530 labeled as positive, the processor 150 may train the deep learning model 500 such that the feature vectors of the antibiotic data 530 become similar to the feature vectors of the corresponding positive control group data 510.

Conversely, in the case of negatively labeled antibiotic data 530, the processor 150 may train the deep learning model 500 such that the feature vectors of the antibiotic data 530 become similar to the feature vectors of the corresponding negative control group data 520.

According to one embodiment of the present invention, by training a deep learning model 500 using feature vectors of data, it is possible to accurately determine whether it is positive or negative without being affected by the difference in time-series electrical characteristic changes between antibiotic data and control group data.

FIG. 6 is a drawing showing the operation flow diagram of an electronic device according to a first embodiment of the present invention.

As described above with reference to S20 of FIG. 3 and FIG. 5, the process of using a deep learning model 500 trained to determine whether antibiotic data of a target microorganism is positive or negative is described.

The processor 150 may extract feature vectors of positive control group data, negative control group data and antibiotic data of a target microorganism, respectively S21. Similarly, for the target microorganism, positive control group data, negative control group data and antibiotic data are prepared as one set under the same conditions (same culture medium, same treatment method, etc.).

The processor 150 may measure the similarity between the feature vectors of the antibiotic data and the feature vectors of the positive control group data and the negative control group data by using the deep learning model 500 (S22). In this case, the algorithm for measuring the similarity may be employed without limitation, such as cosine similarity measurement.

The processor 150 may determine whether the antibiotic data of the target microorganism is positive or negative based on the similarity S23.

For example, if the feature vectors of the antibiotic data have a high similarity to the feature vectors of the positive control group data, the processor 150 may determine that the target microorganism is resistant to the antibiotic. Conversely, if the feature vectors of the antibiotic data have a high similarity to the feature vectors of the negative control group data, the processor 150 may determine that the target microorganism is susceptible to the antibiotic.

According to one embodiment of the present invention, by using a deep learning model 500 trained to make feature vectors similar between data, data whose time-series electrical characteristic change pattern is unclear may be more accurately determined as positive or negative.

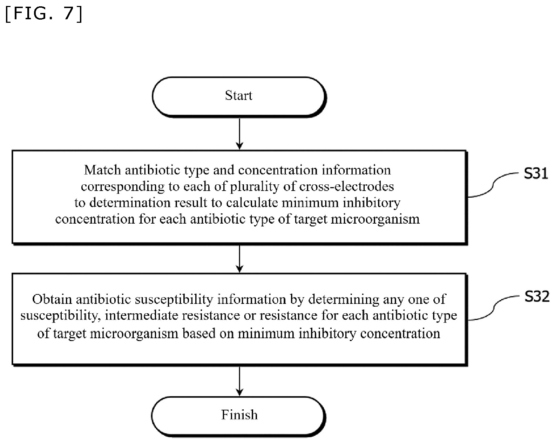

FIG. 7 is a drawing showing the operation flow diagram of an electronic device according to a second embodiment of the present invention.

As described above with reference to S30 of FIG. 3, the process of obtaining antibiotic susceptibility information based on the results determined by the deep learning model 500 is described.

According to one embodiment of the present invention, the processor 150 may calculate a minimum inhibitory concentration of each antibiotic type of the target microorganism by matching the antibiotic type and concentration information corresponding to each of a plurality of cross-electrodes to the determination result S31.

Each cross-electrode of the biosensor 10 corresponds to one channel, and information mapping the type of antibiotic and the concentration of antibiotic to each channel (referred to as antibiotic type and concentration information) may be prepared.

For example, if the target microorganism is *E. coli,* the determination result of determining whether it is positive or negative according to each antibiotic data of *E. coli* using a deep learning model 500 is as shown in Table 1 below. As a result of the determination, if it is positive, it is indicated as P, and if it is negative, it is indicated as N

**[Table 1]**

| CH001 | CH002 | CH003 | CH004 | CH005 | CH006 | CH007 | CH008 | CH009 | CH010 | CH011 | CH012 |
|---|---|---|---|---|---|---|---|---|---|---|---|
| Ampicillin 0.25 µg/mL | Ampicillin 0.5 µg/mL | Ampicillin 1µg/m L | Ampicillin 2µg/m L | Ampicillin 4µg/m L | Ampicillin 8µg/m L | Ampicillin 16 µg/mL | Ampicillin 32 µg/mL | Ertapenem 0.5 µg/mL | Ertapenem 1µg/mL | Ertapenem 2µg/mL | Ertapenem 4µg/mL |
| P | P | P | P | P | P | P | P | N | N | N | N |

| CH01 3 | CH01 4 | CH01 5 | CH01 6 | CH01 7 | CH01 8 | CH01 9 | CH02 0 | CH02 1 | CH02 2 | CH02 3 | CH02 4 |
|---|---|---|---|---|---|---|---|---|---|---|---|
| Mero penem 0.25 µg/mL | Mero penem 0.5 µg/mL | Mero penem 1µg/m L | Mero penem 2µg/m L | Mero penem 4µg/m L | Mero penem 8µg/m L | Mero penem 16 µg/mL | Cefo xitin 2µg/m L | Cefo xitin 4µg/m L | Cefo xitin 8µg/m L | Cefo xitin 16 µg/mL | Cefo xitin 32 µg/mL |
| P | P | N | N | N | N | N | N | N | N | N | N |

| CH02 5 | CH02 6 | CH02 7 | CH02 8 | CH02 9 | CH03 0 | CH03 1 | CH03 2 | CH03 3 | CH03 4 | CH03 5 | CH03 6 |
|---|---|---|---|---|---|---|---|---|---|---|---|
| Cefuroxime 1µg/m L | Cefuroxime 2µg/m L | Cefuroxime 4µg/m L | Cefuroxime 8µg/m L | Cefuroxime 16 µg/mL | Cefuroxime 32 µg/mL | Amoxicillin/ clavulanate 2/1 µg/mL | Amoxicillin/ clavulanate 4/2 µg/mL | Amoxicillin/ clavulanate 8/4 µg/mL | Amoxicillin/ clavulanate 16/8 µg/mL | Amoxicillin/ clavulanate 32/16 µg/mL | Amoxicillin / clavulana te 64/32 µg/mL |
| P | P | P | P | P | P | P | P | P | P | P | P |

| CH037 | CH038 | CH039 | CH040 | CH041 | CH042 | CH043 | CH044 | CH045 | CH046 | CH047 | CH048 |
|---|---|---|---|---|---|---|---|---|---|---|---|
| Amox i cillin 2µg/m L | Amoxi cillin 4µg/m L | Amoxi cillin 8µg/m L | Penici llin 0.12 µg/mL | Penici llin 0.25 µg/mL | Penici llin 0.5 µg/mL | Penici 11 in 1 µg/mL | Penici 11 in 2 µg/mL | Penici 11 in 4 µg/mL | Penici 11 in 8 µg/mL | Penici 11 in 16 µg/mL | Cefepi me 0.5 µg/mL |
| P | P | P | P | P | P | P | P | P | P | P | P |

| CH04 9 | CH05 0 | CH05 1 | CH05 2 | CH05 3 | CH05 4 | CH05 5 | CH05 6 | CH05 7 | CH05 8 | CH05 9 | CH06 0 |
|---|---|---|---|---|---|---|---|---|---|---|---|
| Cefepi me 1 µg/mL | Cefepi me 2 µg/mL | Cefepi me 4 µg/mL | Cefepi me 8 µg/mL | Cefepi me 16 µg/mL | Cefepi me 32 µg/mL | Pipera cillin 8µg/m L | Pipera cillin 16 µg/mL | Pipera cillin 32 µg/mL | Pipera cillin 64 µg/mL | Pipera cillin 128 µg/mL | Cefota xime 0.5 µg/mL |
| N | N | N | N | N | N | P | P | P | P | P | P |

| CH06 1 | CH06 2 | CH06 3 | CH06 4 | CH06 5 | CH06 6 | CH06 7 | CH06 8 | CH06 9 | CH07 0 | CH07 1 | CH07 2 |
|---|---|---|---|---|---|---|---|---|---|---|---|
| Cefota xime 1µg/m L | Cefota xime 2µg/m L | Cefota xime 4µg/m L | Cefota xime 8µg/m L | Cefota xime 16 µg/mL | Cefota xime 32 µg/mL | Ceftaz idime/ clavul anate 1/4 µg/mL | Ceftaz idime/ clavul anate 2/4 µg/mL | Cefota xime/c lavula nate 0.25/4 µg/mL | Cefota xime/c lavula nate 0.5/4 µg/mL | Ceftri axone 0.5 µg/mL | Ceftri axone 1µg/m L |
| P | P | P | P | N | N | P | P | P | P | P | P |

| CH07 3 | CH07 4 | CH07 5 | CH07 6 | CH07 7 | CH07 8 | CH07 9 | CH08 0 | CH08 1 | CH08 2 | CH08 3 | CH08 4 |
|---|---|---|---|---|---|---|---|---|---|---|---|
| Ceftri axone 2µg/m L | Ceftri axone 4µg/m L | Ceftri axone 8µg/m L | Ceftri axone 16 µg/mL | Ceftri axone 32 µg/mL | Oxacil lin 0.25 µg/mL | Oxacil lin 0.5 µg/mL | Cefaz olin 2µg/m L | Cefaz olin 4µg/m L | Cefaz olin 8µg/m L | Cefaz olin 16 µg/mL | Cefaz olin 32 µg/mL |
| P | P | P | P | P | P | P | P | P | P | P | P |

| CH08 5 | CH08 6 | CH08 7 | CH08 8 | CH08 9 | CH09 0 | CH09 1 | CH09 2 | CH09 3 | CH09 4 | CH09 5 | CH09 6 |
|---|---|---|---|---|---|---|---|---|---|---|---|
| Aztre onam 1µg/m L | Aztreo nam 2µg/m L | Aztreo nam 4µg/m L | Aztreo nam 8µg/m L | Aztreo nam 16 µg/mL | Aztreo nam 32 µg/mL | Imipe nem 1µg/m L | Imipe nem 2µg/m L | Imipe nem 4µg/m L | Imipe nem 8µg/m L | Imipe nem 16 µg/mL | Genta micin 2µg/m L |
| P | P | P | N | N | N | P | N | N | N | N | N |

| CH09 7 | CH09 8 | CH09 9 | CH10 0 | CH10 1 | CH10 2 | CH10 3 | CH10 4 | CH10 5 | CH10 6 | CH10 7 | CH10 8 |
|---|---|---|---|---|---|---|---|---|---|---|---|
| Genta micin 4µg/m L | Genta micin 8µg/m L | Genta micin 16 µg/mL | Amika cin 8µg/m L | Amika cin 16 µg/mL | Amika cin 32 µg/mL | Amika cin 64 µg/mL | Strept omyci n 500 µg/mL | Genta micin 1,000 µg/mL | Doxyc ycline 2µg/m L | Doxyc ycline 4µg/m L | Doxyc ycline 8µg/m L |
| N | N | N | N | N | N | N | N | N | N | N | N |

| CH10 9 | CH11 0 | CH11 1 | CH11 2 | CH11 3 | CH11 4 | CH11 5 | CH11 6 | CH11 7 | CH11 8 | CH11 9 | CH12 0 |
|---|---|---|---|---|---|---|---|---|---|---|---|
| Doxycycline 16 µg/mL | Tetracycline 1µg/mL | Tetracycline 2µg/mL | Tetracycline 4µg/mL | Tetracycline 8µg/m L | Tetracycline 16 µg/mL | Tigecycline 0.5 µg/mL | Tigecycline 1µg/mL | Tigecycline 2µg/mL | Tigecycline 4µg/mL | Minocycline 4µg/mL | Minocycline 8µg/mL |
| N | N | N | N | N | N | N | N | N | N | N | N |

| CH12 1 | CH12 2 | CH12 3 | CH12 4 | CH12 5 | CH12 6 | CH12 7 | CH12 8 | CH12 9 | CH13 0 | CH13 1 | CH13 2 |
|---|---|---|---|---|---|---|---|---|---|---|---|
| Minoc ycline 16 µg/mL | Ciprof loxaci n 0.06 µg/mL | Ciprof loxaci n 0.12 µg/mL | Ciprof loxaci n 0.25 µg/mL | Ciprof loxaci n 0.5 µg/mL | Ciprof loxaci n 1µg/m L | Ciprof loxaci n 2µg/m L | Ciprof loxaci n 4 µg/mL | Levofl oxacin 0.12 µg/mL | Levofl oxacin 0.25 µg/mL | Levofl oxacin 0.5 µg/mL | Levofl oxacin 1µg/m L |
| N | P | P | P | P | P | P | P | N | N | N | N |

| CH13 3 | CH13 4 | CH13 5 | CH13 6 | CH13 7 | CH13 8 | CH13 9 | CH14 0 | CH14 1 | CH14 2 | CH14 3 | CH14 4 |
|---|---|---|---|---|---|---|---|---|---|---|---|
| Levofl oxacin 2µg/m L | Levofl oxacin 4µg/m L | Levofl oxacin 8µg/m L | Moxif loxaci n 0.5 µg/mL | Moxif loxaci n 1µg/m L | Moxif loxaci n 2µg/m L | Moxif loxaci n 4µg/m L | Teico planin 8µg/m L | Teico planin 16 µg/mL | Teico planin 32 µg/mL | Vanco mycin 1µg/m L | Vanco mycin 2µg/m L |
| N | N | N | N | N | N | N | P | P | P | P | P |

| CH14 5 | CH14 6 | CH14 7 | CH14 8 | CH14 9 | CH15 0 | CH15 1 | CH15 2 | CH15 3 | CH15 4 | CH15 5 | CH15 6 |
|---|---|---|---|---|---|---|---|---|---|---|---|
| Vanco mycin 4µg/m L | Vanco mycin 8µg/m L | Vanco mycin 16 µg/mL | Vanco mycin 32 µg/mL | Line zolid 2µg/m L | Line zolid 4µg/m L | Line zolid 8µg/m L | ICR 1/0.5 µg/mL | ICR 4/0.5 µg/mL | Erythr omyci n 0.25 µg/mL | Erythr omyci n 0.5 µg/mL | Erythr omyci n 1µg/m L |
| P | P | P | P | P | P | P | P | P | P | P | P |

| CH15 7 | CH15 8 | CH15 9 | CH16 0 | CH16 1 | CH16 2 | CH16 3 | CH16 4 | CH16 5 | CH16 6 | CH16 7 | CH16 8 |
|---|---|---|---|---|---|---|---|---|---|---|---|
| Erythr omyci n 2µg/m L | Erythr omyci n 4µg/m L | Erythr omyci n 8µg/m L | Clinda mycin 0.25 µg/mL | Clinda mycin 0.5 µg/mL | Clinda mycin 1µg/m L | Clinda mycin 2µg/m L | Clinda mycin 4µg/m L | Trimet hopri m/sulf ameth oxazole 0.5/9. 5 µg/mL | Trimet hopri m/sulf ameth oxazole 1/19 µg/mL | Trimet hopri m/sulf ameth oxazole 2/38 µg/mL | Trimet hopri m/sulf ameth oxazole 4/76 µg/mL |
| P | P | P | P | P | P | P | P | N | N | N | N |

| CH16 9 | CH17 0 | CH17 1 | CH17 2 | CH17 3 | CH17 4 | CH17 5 | CH17 6 | CH17 7 | CH17 8 | CH17 9 | CH18 0 |
|---|---|---|---|---|---|---|---|---|---|---|---|
| Trime thopri m/sulf ameth oxazol e 8/152 µg/mL | Nitrof uranto in 32 µg/mL | Nitrof uranto in 64 µg/mL | Nitrof uranto in 128 µg/mL | Mupir ocin 128 µg/mL | Mupir ocin 256 µg/mL | Nitrox oline 16 µg/mL | Nitrox oline 32 µg/mL | Colisti n 1µg/m L | Colisti n 2µg/m L | Colisti n 4µg/m L | Colisti n 8µg/m L |
| N | N | N | N | N | N | N | N | N | N | N | N |

| CH18 1 | CH18 2 | CH18 3 | CH18 4 | CH18 5 | CH18 6 | CH18 7 | CH18 8 | CH18 9 | CH19 0 | CH19 1 | CH19 2 |
|---|---|---|---|---|---|---|---|---|---|---|---|
| Positive Control | | | | | | Negative Control | | | | | |

Looking at Table 1, channels 1 to 8 represent the determination results of antibiotic data obtained by treating with ampicillin among antibiotics at different concentrations (0.25, 0.5, 1, 2, 4, 8, 16, 32 µg/mL). The corresponding *E. coli* was determined to be positive for ampicillin up to a concentration of 32 µg/mL. Therefore, although the minimum inhibitory concentration of the corresponding *E. coli* for ampicillin is not known exactly, it may be calculated to exceed at least a concentration of 32 µg/mL.

In addition, channels 61 to 66 in Table 1 represent the results of determining antibiotic data obtained by treating with Cefotaxime at different concentrations (1, 2, 4, 8, 16, 32 µg/mL). The *E. coli* was determined to be positive up to a concentration of 8 (µg/mL), but negative at concentrations of 16 µg/mL and 32 µg/mL. Therefore, the minimum inhibitory concentration of the *E. coli* against Cefotaxime may be calculated to be 16 µg/mL.

According to one embodiment of the present invention, the processor 150 may obtain antibiotic susceptibility information by determining any one of the susceptibility (S), intermediate resistance (I) and resistance (R) of the target microorganism to the antibiotic type based on the minimum inhibitory concentration S32.

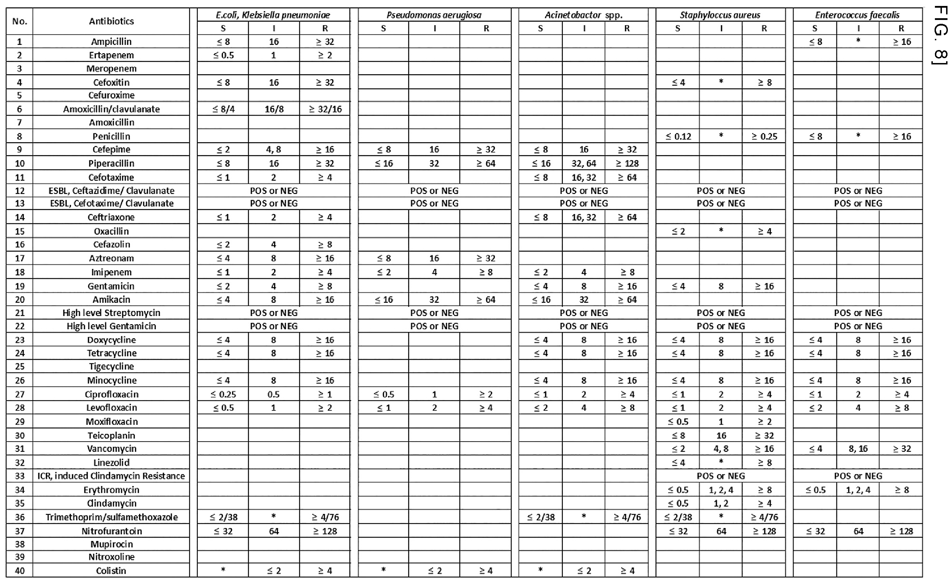

In this case, the processor 150 may determine any one of susceptibility, intermediate resistance and resistance for each type of antibiotic by applying the antibiotic susceptibility test performance standards (e.g., CLSI guidelines, etc.) of the target microorganism. The antibiotic susceptibility test performance criteria are established for each microorganism, and an example of the CLSI guidelines is shown in FIG. 8.

Table 2 below is an example of antibiotic susceptibility information obtained based on the results in Table 1 above.

**[Table 2]**

| **No.** | **Antibiotics** | **MIC (µg/mL)** | **S/I/R** |
|---|---|---|---|
| 1 | Ampicillin, Amp | > 32 | R |
| 2 | Ertapenem, Erta | ≤ 0.5 | S |
| 3 | Meropenem, Mero | 1 | * |
| 4 | Cefoxitin, Ctin | ≤ 2 | S |
| 5 | Cefuroxime, Cefu | > 32 | * |
| 6 | Amoxicillin-clavulanate, Amx-c | > 64/32 | R |
| 7 | Amoxicillin, Amx | > 8 | * |
| 8 | Penicillin, Peni | > 16 | * |
| 9 | Cefepime, Pime | 1 | S |
| 10 | Piperacillin, Pipe | > 128 | R |
| 11 | Cefotaxime, Taxi | 16 | R |
| 12 | Ceftazidime-Clavulanate, Dime-c | Pos. | |
| 13 | Cefotaxime-Clavulanate, Taxi-c | Pos. | |
| 14 | Ceftriaxone, Axone | > 32 | R |
| 15 | Oxacillin, Oxa | > 0.5 | * |
| 16 | Cefazolin, Zolin | > 32 | R |
| 17 | Aztreonam, Azt | 8 | I |
| 18 | Imipenem, Imi | 2 | I |
| 19 | Gentamicin, Gen | ≤ 2 | S |
| 20 | Amikacin, Amk | ≤ 8 | I |
| 21 | High level Streptomycin, HLSM | Neg. | |
| 22 | High level Gentamicin, HLGM | Neg. | |
| 23 | Doxycycline, Doxy | ≤ 2 | S |
| 24 | Tetracycline, Tetra | ≤ 1 | S |
| 25 | Tigecycline, Tige | ≤ 0.5 | * |
| 26 | Minocycline, Mino | ≤ 4 | S |
| 27 | Ciprofloxacin, Cipro | > 4 | R |
| 28 | Levofloxacin, Levo | ≤ 0.12 | S |
| 29 | Moxifloxacin, Moxi | ≤ 0.5 | * |
| 30 | Teicoplanin, Tei | > 32 | * |
| 31 | Vancomycin, Van | > 32 | * |
| 32 | Linezolid, Line | > 8 | * |
| 33 | Induced Clindamycin Resistance, ICR | Pos. | |
| 34 | Erythromycin, Ery | > 8 | * |
| 35 | Clindamycin, Clinda | > 4 | * |
| 36 | Trimethoprim/sulfamethoxazole, Tri/Sul | ≤ 0.5/9.5 | S |
| 37 | Nitrofurantoin, Nifur | ≤ 32 | S |
| 38 | Mupirocin, Mupi | ≤ 128 | * |
| 39 | Nitroxoline, Nirox | ≤ 16 | * |
| 40 | Colistin, Col | ≤ 1 | I |

Looking at Table 2, the corresponding *E. coli* was determined to be positive for ampicillin up to a concentration of 32 µg/mL. The processor 150 may determine that the corresponding *E. coli* is resistant (R) to ampicillin based on the guidelines of FIG. 8.

In addition, since the minimum inhibitory concentration of the corresponding *E. coli* to cytotoxicity is 16 µg/mL, the processor 150 may determine that the corresponding *E. coli* is resistant (R) to cytotoxicity based on the guidelines of FIG. 8.

The national research and development project that supported the present application is as follows.
[Project Identification Number] 2420005260
[Project Number] RS-2024-00442440
[Name of Ministry] Ministry of SMEs and Startups
[Name of Project Management (Specialized) Institution] Korea Technology and Information Promotion Agency for SMEs
[Title of Research Project] Small and Medium Enterprise Technology Innovation Development Project Export-Oriented (Export)
[Title of Research Task] Commercialization of Rapid, Fully Automatic Antibiotic Susceptibility Testing System Using New Technology in Electrical Capacity Measurement
[Contribution Ratio] 1/1
[Name of Project Performance Institution] PROTIA INC.
[Research Period] August 1, 2024 to July 31, 2028

## Claims

1. An electronic device for performing an antibiotic susceptibility test of a microorganism, comprising:
a processor configured to: obtain antibiotic data obtained by measuring changes in electrical characteristics according to antibiotic treatment of a target microorganism;
input positive control group data, negative control group data and antibiotic data of the target microorganism into a deep learning model trained to determine whether the antibiotic data is positive or negative by using positive control group data, negative control group data and antibiotic data of a microorganism, thereby determining whether antibiotic data of the target microorganism is positive or negative; and
use the determination result so as to identify antibiotic susceptibility information about the target microorganism according to antibiotic type and concentration.

2. The electronic device of claim 1, wherein the deep learning model extracts feature vectors of positive control group data, negative control group data and antibiotic data of a microorganism, respectively, and learns to make feature vectors of the antibiotic data similar to feature vectors of the negative control group data or the positive control group data based on labeling of the antibiotic data as positive or negative.

3. The electronic device of claim 2, wherein the processor extracts feature vectors of positive control group data, negative control group data and antibiotic data of the target microorganism, measures similarity between feature vectors of the antibiotic data and feature vectors of the positive control group data and the negative control group data by using the deep learning model, and determines whether antibiotic data of the target microorganism is positive or negative based on the similarity.

4. The electronic device of claim 1, wherein the processor obtains the antibiotic data through a biosensor comprising a plurality of cross-electrodes that are implemented to measure in real time changes in electrical characteristics according to the proliferation of a microorganism in response to antibiotic treatment.

5. The electronic device of claim 4, wherein the processor calculates a minimum inhibitory concentration (MIC) for each antibiotic type of the target microorganism by matching antibiotic type and concentration information corresponding to each of the plurality of cross-electrodes with the determination result.

6. The electronic device of claim 5, wherein the processor determines any one of susceptibility, intermediate resistance and resistance of the target microorganism to each antibiotic type based on the minimum inhibitory concentration, thereby obtaining the antibiotic susceptibility information.

7. A method for testing antibiotic susceptibility of a microorganism performed by an electronic device, the method comprising:
obtaining antibiotic data by measuring changes in electrical characteristics of a target microorganism according to antibiotic treatment;
inputting positive control group data, negative control group data and antibiotic data of the target microorganism into a deep learning model trained to determine whether antibiotic data of a microorganism is positive or negative by using positive control group data, negative control group data and antibiotic data of the microorganism, thereby determining whether antibiotic data of the target microorganism is positive or negative; and
using the determination result so as to identify antibiotic susceptibility information about the target microorganism according to an antibiotic type and concentration.
